(19) Europäisches Patentamt
European Patent Office
Office européen des brevets

(11) **EP 4 252 785 A1**

(12) **EUROPEAN PATENT APPLICATION**

(43) Date of publication:
**04.10.2023 Bulletin 2023/40**

(21) Application number: **23163617.6**

(22) Date of filing: **23.03.2023**

(51) International Patent Classification (IPC):
**A61L 9/20** $^{(2006.01)}$    **A61N 5/06** $^{(2006.01)}$

(52) Cooperative Patent Classification (CPC):
**A61N 5/0624; A61L 9/20;** A61L 2209/14;
A61N 2005/0667

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.03.2022 JP 2022054829**

(71) Applicant: **Ushio Denki Kabushiki Kaisha Tokyo 100-8150 (JP)**

(72) Inventor: **YAGYU, Hideaki Tokyo, 100-8150 (JP)**

(74) Representative: **Tomerius, Isabel Lang & Tomerius Patentanwaltspartnerschaft mbB Rosa-Bavarese-Strasse 5 80639 München (DE)**

(54) **ULTRAVIOLET IRRADIATION DEVICE**

(57)    An ultraviolet irradiation device (1) includes a light source (30) that emits light showing light intensity in a first wavelength band of 200 nm to 235 nm and a second wavelength band of 240 nm to 280 nm; an optical filter (40) that suppresses the light intensity in the second wavelength band and has transmittance characteristics according to an incident angle of the light emitted from the light source (30). A first/ second angular distribution of light is obtained from a spectrum for each light distribution angle of the light emitted from the optical filter (40). The first angular distribution of light has a first angle of a light beam showing maximum intensity, the first angle substantially matching with a second angle of a light beam showing maximum intensity in the second angular distribution of light.

Fig. 4

**Description**

BACKGROUND OF THE INVENTION

Field of the Invention

The present invention relates to an ultraviolet irradiation device.

Description of the Related Art

In recent years, the influence of ultraviolet light on a person has been researched, and it has been proved that ultraviolet light has characteristics of being easily absorbed in the skin surface layer and the corneal epithelium and being improved in safety with a decrease in wavelength. Therefore, there has been studied a technique of inactivating pathogens such as bacteria, fungi, and viruses that exist in a space or the like in an environment in which the person is present by irradiating the environment with ultraviolet light having a relatively short wavelength. For example, Patent Document 1 discloses an ultraviolet irradiation device that can inactivate pathogens by emitting ultraviolet light into a space in which a person is present, the ultraviolet light being in a wavelength range having extremely low harmfulness to a person and being able to inactivate the pathogens.

Incidentally, at the time of applying the ultraviolet light to an environment including a person, there is a case where an ultraviolet irradiation dose is required to be reduced. For example, the ultraviolet irradiation dose per day (8 hours) for a person is specified to be a threshold limit value (TLV) or less by American Conference of Governmental Industrial Hygienists (ACGIH) or JIS Z 8812 (Japanese Industrial Standards related to Measuring methods of eye-hazardous ultraviolet radiation).

Prior Art Document

Patent Document

Patent Document 1: JP-B2-6908172

SUMMARY OF THE INVENTION

Although it has been confirmed that the ultraviolet light having a relatively short wavelength that can be emitted to the environment including a person has extremely low harmfulness to people and animals, in order to obtain high safety and security, the emission intensity or the actual irradiation time of the ultraviolet light is desired to be set so that the ultraviolet irradiation dose satisfies the regulation of the threshold limit value (TLV) described above. On the other hand, in order to enhance the inactivation capability of the ultraviolet irradiation device, it is effective to increase the ultraviolet irradiation dose.

An object of the present invention is to provide an improved ultraviolet irradiation device that easily improves pathogens inactivation capability while ensuring high safety for people and animals.

An ultraviolet irradiation device according to an aspect of the present invention includes:

a light source that emits light showing light intensity in a first wavelength band of 200 nm to 235 nm and a second wavelength band of 240 nm to 280 nm and having at least a part of a main emission wavelength band belonging to the first wavelength band;

an optical filter that suppresses the light intensity in the second wavelength band and has transmittance characteristics according to an incident angle of the light emitted from the light source, in which,

regarding a first angular distribution of light obtained from a spectrum for each light distribution angle of the light emitted from the optical filter by integrating the light intensity with a wavelength and showing an integrated value for each light distribution angle, the light intensity being in a wavelength band where the main emission wavelength band overlaps the first wavelength band, and

regarding a second angular distribution of light obtained from the spectrum for each light distribution angle by integrating the light intensity in the second wavelength band with a wavelength and showing an integrated value for each light distribution angle,

the first light distribution angle distribution has a first angle of a light beam showing maximum intensity, the first angle substantially matching with a second angle of a light beam showing maximum intensity in the second angular distribution of light.

First, definitions of terms used in the present specification are described.

In the present specification, "showing intensity" means that the emitted light from the light source is detected by a measuring instrument such as a spectroscope (spectrophotometer).

In the present specification, the optical filter that "suppresses light intensity" means that, in a case where light intensity of the incident light incident on the optical filter at an incident angle of 0° is compared with light intensity of the emitted light emitted from the optical filter, the optical filter is one that reduces the light intensity of the emitted light in a target wavelength region with respect to the light intensity of the incident light. The optical filter that "suppresses light intensity" is typically an optical filter in which the light intensity of the emitted light is 50% or less with respect to the light intensity of the incident light in a case where the light intensities are compared with each other in terms of an integral value of the entire target wavelength range, and more preferably an optical filter in which the light intensity of the emitted light is 30% or less with respect to the light intensity of the incident light. The optical filter that "suppresses light intensity" may be an optical filter in which a light intensity ratio in the second wavelength band to the maximum intensity of the light belonging to the first wavelength band in the emitted light is lower than the light intensity ratio in the incident light.

In the present specification, the phrase "two angles of a light beam substantially match with each other" includes a case where the angles are different within ±15 degrees in addition to a case where the angles completely match with each other.

The reason of the above-described ultraviolet irradiation device being means for solving the above-described problems is described. A spectrum emitted by a light source used in the ultraviolet irradiation device shows light intensity in a first wavelength band of 200 nm to 235 nm and a second wavelength band of 240 nm to 280 nm, and at least a part of the main emission wavelength band belongs to the first wavelength band. In the present specification, in a case of being simply described as a "spectrum", the "spectrum" refers to a spectral spectrum representing light intensity corresponding to a wavelength. In the present specification, a "main emission wavelength band" refers to a wavelength band across which a spectrum of light emitted from the light source shows light intensity of a certain ratio or more to the maximum intensity (for example, 10% or more of the maximum intensity). Further, the "main emission wavelength band" may be determined based on a wavelength showing the maximum intensity in the first wavelength band. For example, a wavelength within ± 5 nm of the maximum intensity in the first wavelength band may be determined as the "main emission wavelength band"

The light belonging to the first wavelength band of 200 nm to 235 nm is light having a function of inactivating pathogens and having extremely low harmfulness to people and animals. In the present specification, a term "pathogens" includes germs such as bacteria and fungi (mold), and viruses. "Inactivation" is a concept of killing pathogens or causing pathogens to lose their infectivity or toxicity. In the present specification, the light belonging to the first wavelength band of 200 nm to 235 nm is used for the inactivation purpose. Hereinafter, there is a case where the light in the first wavelength band of 200 nm to 235 nm is referred to as "target light".

The light belonging to the second wavelength band of 240 nm to 280 nm is light that may adversely affect people and animals. In the present specification, the light belonging to the second wavelength band is the light not desirably emitted but the light that is inevitably emitted due to the nature of the light source. Hereinafter, there is a case where the light in the second wavelength band of 240 nm to 280 nm is referred to as "harmful light".

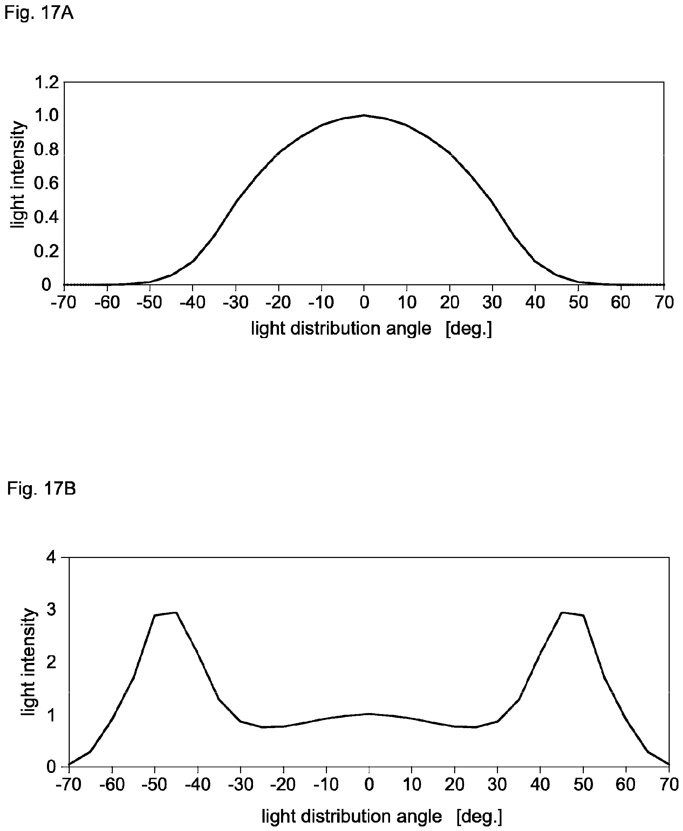

Fig. 17A is a graph showing an angular distribution of light of target light emitted from a conventional ultraviolet irradiation device by simulation. Fig. 17B is a graph showing an angular distribution of light of harmful light emitted from the conventional ultraviolet irradiation device by simulation. In the simulation, a KrCl excimer lamp in which krypton (Kr) and chlorine (Cl) are sealed in a light-emitting tube as light emission gas is used as a light source of the ultraviolet irradiation device.

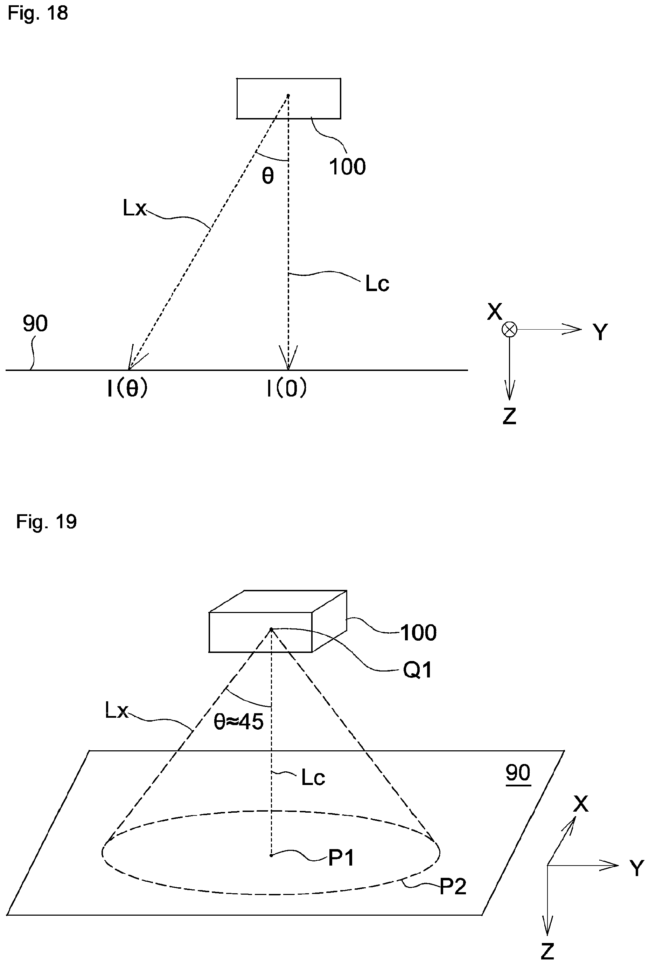

The angular distribution of light is the light intensity distribution for each light distribution angle and is represented by a graph showing a relative value of light intensity for each light distribution angle. The light distribution angle and the light intensity are described. Fig. 18 is a diagram explaining a light distribution angle and light intensity $I(\theta)$. As shown in Fig. 18, the light distribution angle is represented by an angle formed between an arbitrary light beam Lx emitted from an ultraviolet irradiation device 100 and an optical axis Lc of a light flux emitted from the ultraviolet irradiation device 100. The light intensity $I(\theta)$ is light intensity on an arbitrary irradiated surface 90. The light intensity $I(\theta)$ varies depending on the light distribution angle. The light intensity of the light beam on the optical axis Lc at $\theta = 0$ is represented by $I(0)$. The light intensity $I(\theta)$ is expressed by a relative value with respect to $I(0)$ which is 1. Because the light intensity $I(\theta)$ is paraphrased as relative irradiance, there is a case where the light intensity $I(\theta)$ is referred to as "relative irradiance" in the present specification.

In the angular distribution of light shown in each of Figs. 17A and 17B, the horizontal axis represents the light distribution angle (deg.), and the vertical axis represents the light intensity $I(\theta)$. From the graph in Fig. 17A, it can be seen that the light intensity of the target light is maximum when the light distribution angle is 0 degrees, and decreases as the absolute value of the light distribution angle increases. From the graph Fig. 17B, it can be seen that the light intensity of the harmful light is maximum when the light distribution angle is around +45 degrees or -45 degrees. The reason of the light distribution angle showing the maximum intensity being different between the target light and the harmful light is described later.

Fig. 19 is a perspective view showing respective positions of the maximum intensity of the target light and the maximum intensity of the harmful light emitted from the ultraviolet irradiation device 100. Based on Figs. 17A and 17B, the maximum relative irradiance of the target light appears at a position P1 in Fig. 19. The maximum relative irradiance of the harmful light appears at a position P2 in Fig. 19. Note that, in the present specification, because it is assumed that the light distribution angle indicating the maximum intensity around the optical axis Lc does not vary, the position P2 is represented as a circle. However, actually, because the light distribution angle indicating the maximum intensity sometimes fluctuate around the optical axis Lc, the position P2 is not always represented by a circle.

Incidentally, the threshold limit value described above (hereinafter, sometimes referred to as "TLV") is defined for each wavelength. In addition, the TLV is reviewed over time with the progress of safety research for each wavelength of light. For example, in the currentACGIH, the TLV of light having a wavelength of 222 nm is 22 mJ/cm$^2$ per day (8 hours). However, the TLV of light having a wavelength of 222 nm is expected to be relaxed in consideration of safety to the human body.

The present inventor has considered a case of increasing the emission intensity or the actual irradiation time of the ultraviolet irradiation device according to the relaxation of the TLV. If the emission intensity or the actual irradiation time can be increased while the relaxation of the TLV is satisfied, the inactivation capability can be enhanced while high safety for humans and animals is secured. In the course of such studies, the present inventors have found that a problem that has not been assumed conventionally occurs when the emission intensity or the actual irradiation time is increased.

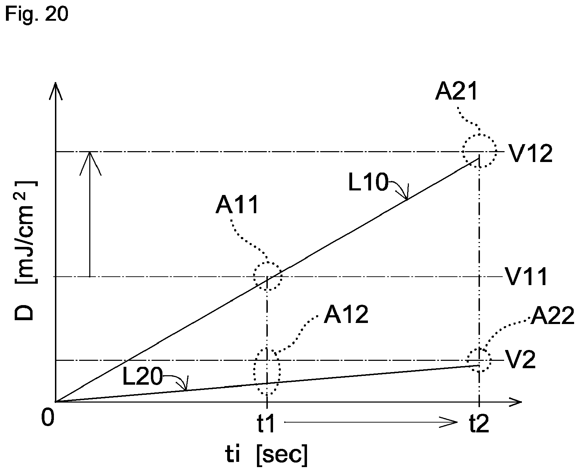

This problem is described with reference to Fig. 20. Fig. 20 is a graph showing the relationship between an irradiation time ti (horizontal axis) and a daily irradiation dose D (vertical axis) of target light L10 and harmful light L20. The ultraviolet irradiation device may perform not only the operation of continuously performing irradiation with the ultraviolet light but also the operation of intermittently repeating irradiation and non-irradiation in one day. The irradiation time ti means the total sum of periods of irradiation time in one day (integrated time). The longer the irradiation time ti (unit: sec), the larger the irradiation dose D (unit: mJ/cm$^2$).

It is assumed that, conventionally, the TLV of the target light L10 is defined to be V11 (mJ/cm$^2$) and the TLV of the harmful light L20 is defined to be V2 (mJ/cm$^2$). Conventionally, the irradiation time is set to t1 (sec) in order for the irradiation dose D of the target light L10 not to exceed V11 (mJ/cm$^2$). That is, only a region A11 has been focused. Because the irradiation dose of the harmful light L20 does not reach V2 (mJ/cm$^2$) at all, if only the region A11 is focused, a region A12 does not need to be focused.

Here, a case is studied where the TLV of the target light is relaxed and the irradiation dose can be set higher from the conventional V11 (mJ/cm$^2$) to a new V12 (mJ/cm$^2$). As a result of studying that the irradiation time is to be extended to t2 (sec) according to the relaxation of the TLV, it has been found that the irradiation dose of the harmful light L20, which has not been necessary to be considered heretofore, approaches V2 (mJ/cm$^2$) which is the TLV of the harmful light L20 (see a region A22).

In that case, in order to set the irradiation time according to the relaxation of the TLV, it is necessary not only to prevent the irradiation dose of the target light L10 from exceeding V12 (mJ/cm$^2$) (see the region A21) but also to prevent the irradiation dose of the harmful light L20 from exceeding V2 (mJ/cm$^2$) which is the TLV of the harmful light (see the region A22).

As shown in Fig. 19, the position P1 irradiated with the light beam indicating the maximum intensity of the target light is different from the position P2 irradiated with the light beam indicating the maximum intensity of the harmful light. Therefore, when the irradiation dose D according to the relaxation of the TLV is set, it is necessary to monitor the position P1 and the position P2 in order to prevent the doses of the target light and the harmful light from exceeding the respective TLVs. In particular, when the light distribution angle indicating the maximum intensity of the harmful light is unknown, first, an effort of measuring the light distribution angle indicating the maximum intensity is further required. Note that, although the setting of the irradiation time according to the relaxation of the TLV has been described above, this applies similarly to the setting of the emission intensity according to the relaxation of the TLV.

As described at the beginning of the section "SUMMARY OF THE INVENTION", as a result of earnest research, the present inventor has found that an ultraviolet irradiation device is designed such that a light distribution angle (first angle) of the light beam having the maximum intensity in a first angular distribution of the target light substantially matches with a light distribution angle (second angle) of the light beam having the maximum intensity in a second angular distribution of the harmful light. As a result, when the irradiation dose D according to the relaxation of the TLV is set, the irradiation doses D of both the target light and the harmful light can be monitored at the same position. As a result, the adjustment of the ultraviolet irradiation device according to the relaxation of the TLV becomes simple.

An example of a method of deriving the first angular distribution and the second angular distribution of the light emitted from the optical filter in the ultraviolet irradiation device is described in detail in the section of "DETAILED DESCRIPTION OF THE PREFERRED EMBODIMENTS".

Both the first angle and the second angle may be between -10 degrees and +10 degrees. Both the first angle and the second angle may be between -5 degrees and +5 degrees. Because both the first angle and the second angle approach 0 degrees, the positions (P1, P2) where the light intensity is monitored can be set in the vicinity of the optical axis Lc of the emitted light. As a result, the adjustment of the ultraviolet irradiation device according to the relaxation of the TLV becomes simple.

In the first angular distribution, the light intensity of the light beam having a light distribution angle of 40 degrees may be smaller than the light intensity of the light beam having the first angle, and the light intensity of the light beam having a light distribution angle of 70 degrees may be smaller than the light intensity of the light beam having a light distribution angle of 40 degrees, and in the second angular distribution, the light intensity of the light beam having a light distribution angle of 40 degrees may be smaller than the light intensity of the light beam having the second angle, and the light intensity of the light beam having a light distribution angle of 70 degrees may be smaller than the light intensity of the light beam having a light distribution angle of 40 degrees. This indicates that the light intensity of the target light and the light intensity of the harmful light both decrease as the absolute value of the light distribution angle gradually increases. When the light distribution angle is large, the distance from the ultraviolet irradiation device is usually large on the irradiated plane. Therefore, the relative irradiance of the target light and the harmful light tends to decrease as the distance from the ultraviolet irradiation device increases, and as a result, the adjustment of the ultraviolet irradiation device according to the relaxation of the TLV becomes simple.

In a spectrum of light emitted from the optical filter, regarding a first integrated light intensity obtained by integrating light intensity of a wavelength band in which the main emission wavelength band overlaps the first wavelength band and a second integrated light intensity obtained by integrating light intensity in the second wavelength band, the second integrated light intensity may be 1.0% or less of the first integrated light intensity. The second integrated light intensity may be 0.1% or less of the first integrated light intensity. By suppressing the second integrated light intensity representing the light intensity of the entire harmful light, the ultraviolet irradiation dose can be increased while the TLV is maintained.

Further, the optical filter may include, at a subsequent stage, a diffusion part that diffuses the emitted light from the optical filter.

The optical filter may have an average transmittance for light in a wavelength band in which the main emission wavelength band overlaps the first wavelength band, the average transmittance showing a decreasing tendency as the incident angle on the optical filter increases from 20 degrees to 60 degrees, and

the optical filter may have the average transmittance for the light in the second wavelength band, the average transmittance showing an increasing tendency as the incident angle on the optical filter increases from 30 degrees to 60 degrees.

Note that a method of deriving the average transmittance for each incident angle in a specific wavelength band included in the optical filter is described later.

There can be provided the improved ultraviolet irradiation device that can easily improve pathogen inactivation capability while securing safety for humans and animals.

The provision of the ultraviolet irradiation device accords with Goal 3 "Ensure healthy lives and promote well-being for all at all ages" included in the sustainable development goals (SDGs) led by the United Nations and will contribute greatly to Target 3.3 "By 2030, end the epidemics of AIDS, tuberculosis, malaria and neglected tropical diseases and combat hepatitis, water-borne diseases and other communicable diseases".

BRIEF DESCRIPTION OF THE DRAWINGS

Fig. 1 is a diagram showing an ultraviolet irradiation device according to an embodiment;
Fig. 2 is a diagram of the ultraviolet irradiation device in Fig. 1 as viewed from the +Z side;
Fig. 3 shows an example of a spectrum of ultraviolet light emitted from a light source;
Fig. 4 is a cross-sectional view of the ultraviolet irradiation device in Fig. 1 as viewed in the X direction;
Fig. 5A shows an average transmittance of the optical filter when an incident angle of the ultraviolet light is made different;
Fig. 5B shows an average transmittance of the optical filter when the incident angle of the ultraviolet light is made different;
Fig. 6 is a diagram for explaining the incident angle of light incident on the optical filter;
Fig. 7A shows a first angular distribution of emitted light of the ultraviolet irradiation device of the present embodiment;
Fig. 7B shows a second angular distribution of the emitted light of the ultraviolet irradiation device of the present embodiment;
Fig. 8 shows a transmission spectrum of an optical filter alone for each incident angle;
Fig. 9 shows an example of a measurement method of obtaining a transmission spectrum $T(\lambda,\theta)$ for each incident angle;
Fig. 10 shows a transmission spectrum of the emitted light of the optical filter for each incident angle;

Fig. 11 shows incident angle characteristics of the optical filter in a second wavelength band;

Fig. 12 shows an example of a measurement method of obtaining the radiation intensity of the emitted light for each light distribution angle;

Fig. 13 shows an angular distribution of the emitted light from an ultraviolet irradiation device without an optical filter;

Fig. 14 shows an angular distribution of light wavelength-integrated with harmful light in the present embodiment;

Fig. 15 shows an angular distribution of light wavelength-integrated with a part of target light in the present embodiment;

Fig. 16 shows a modification example of the ultraviolet irradiation device;

Fig. 17A shows a first angular distribution of emitted light of a conventional ultraviolet irradiation device;

Fig. 17B shows a second angular distribution of the emitted light of the conventional ultraviolet irradiation device;

Fig. 18 is a diagram for explaining a light distribution angle and light intensity;

Fig. 19 is a perspective view showing a position where the maximum relative irradiance of each of the target light and the harmful light is measured; and

Fig. 20 is a diagram showing a relationship between an irradiation time and an irradiation dose for each of target light and harmful light.

## DETAILED DESCRIPTION OF THE PREFERRED EMBODIMENTS

The drawings are shown using an XYZ coordinate system as appropriate. The specification is described with reference to the XYZ coordinate system as appropriate. In describing directions in the present specification, in the case of distinguishing whether the direction is positive or negative, the positive or negative symbol is added, such as the "+X direction" or the "-X direction". In the case where there is no need to distinguish between positive and negative directions, the direction is simply described as the "X direction". Namely, in the present specification, in the case where the direction is simply described as the "X direction", both "+X direction" and "-X direction" are included. The similar applies to the Y direction and the Z direction.

[Outline of ultraviolet irradiation device]

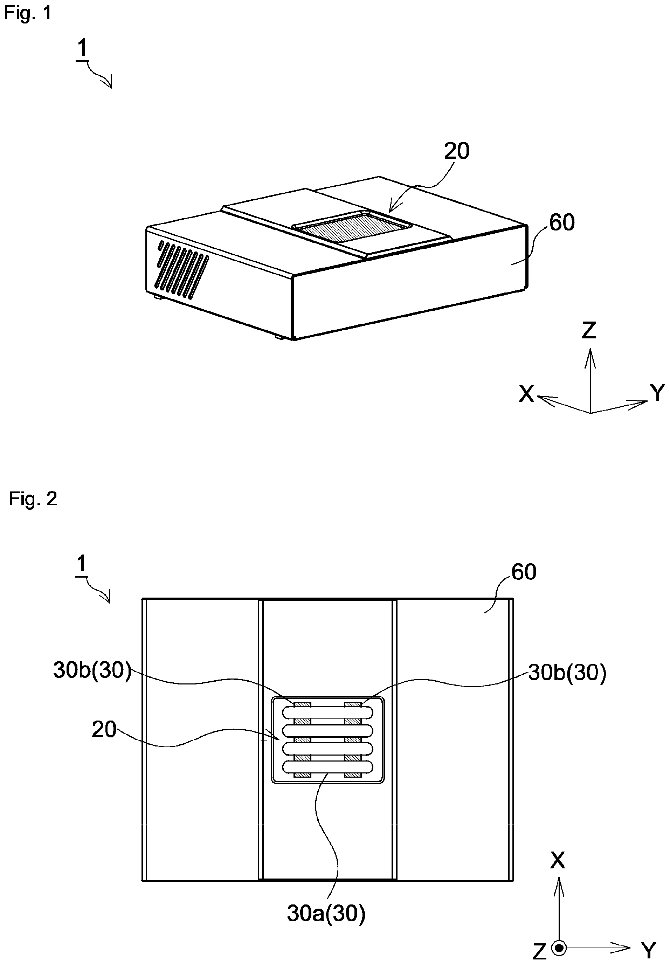

An outline of an embodiment of an ultraviolet irradiation device is described with reference to Fig. 1. Fig. 1 is a diagram schematically showing an external appearance of an ultraviolet irradiation device 1 of the embodiment, and Fig. 2 is a drawing of the ultraviolet irradiation device 1 in Fig. 1 as viewed from the +Z side. As shown in Fig. 2, the ultraviolet irradiation device 1 of the present embodiment includes a casing 60 and a light source 30 housed inside the casing 60.

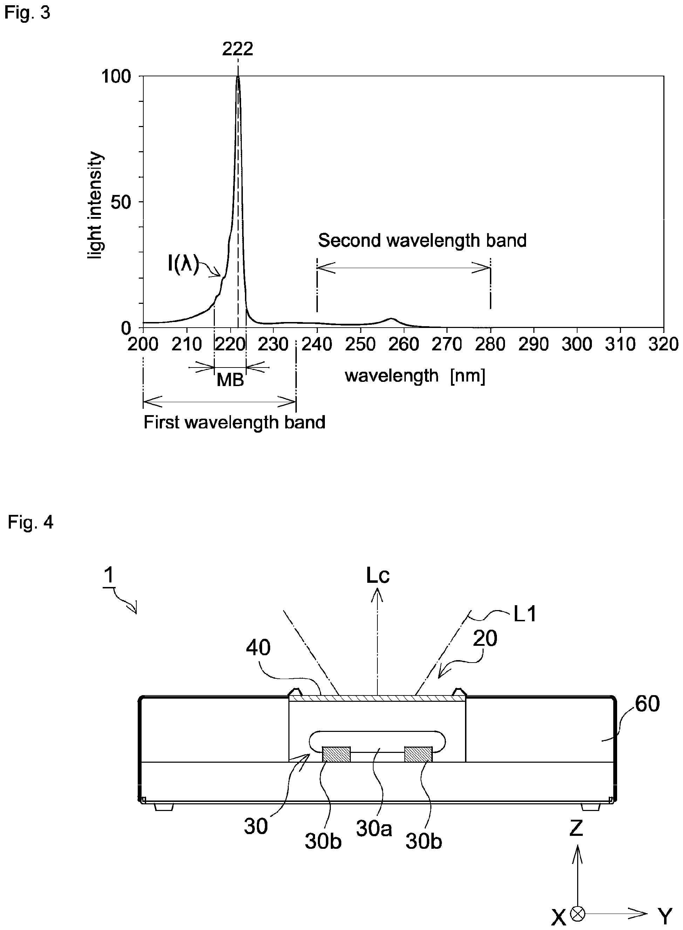

As shown in Fig. 2, the light source 30 of the present embodiment is an excimer lamp including a plurality of light-emitting tubes 30a and a pair of electrodes 30b. A direction in which the plurality of light-emitting tubes 30a is aligned is defined as an X direction. A direction in which the light-emitting tubes 30a extends is defined as a Y direction. A direction orthogonal to the X direction and the Y direction is a Z direction. As shown in Fig. 4 described later, the plurality of light-emitting tubes 30a are placed on the pair of electrodes 30b. The ultraviolet light emitted from the light-emitting tubes 30a is extracted from a light extraction part 20 to the outside of the casing 60.

Fig. 3 is a graph showing an example of a spectrum of ultraviolet light L1 emitted from the light source 30. The light source 30 of the present embodiment is an excimer lamp in which krypton (Kr) gas and chlorine (Cl) gas are sealed as light emission gas G1 in the light-emitting tube 30a. When a voltage is applied between the electrodes (30b, 30b), the ultraviolet light L1 having a wavelength showing the maximum intensity of 222 nm as shown in Fig. 3 is emitted. A wavelength at 222 nm, which is the wavelength showing the maximum intensity, is in a first wavelength band (200 nm to 235 nm).

The ultraviolet light emitted from the light source 30 exhibits a spectrum that has a main emission wavelength band MB between 216 nm or more and 223 nm or less. The main emission wavelength band MB is a wavelength band showing light intensity of 10% or more of the maximum intensity in the light source 30.

As shown in Fig. 3, the light source 30 emits light in a second wavelength band of 240 nm to 280 nm although the emission intensity thereof is very low. Because the light in the second wavelength band is the harmful light, the light in the second wavelength band is restricted by an optical filter to be described later, and the harmful light is prevented from leaking out of the casing 60 of the ultraviolet irradiation device 1.

In the present embodiment, the excimer lamp in which Kr gas and Cl gas are sealed is employed as the light source 30, but the present invention is not limited thereto. An excimer lamp filled with another gas (for example, an excimer lamp filled with Kr gas and Br gas and having the maximum intensity in the vicinity of 207 nm) may be adopted as the light source 30. In addition, a solid state light source such as a light-emitting diode (LED) may be adopted as the light source 30.

In the case of the excimer lamp in which Kr gas and Cl gas are sealed, the entire main emission wavelength band MB belongs to the first wavelength band of 200 nm to 235 nm. However, the entire main emission wavelength band MB may not be located in the first wavelength band. At least a part of the main emission wavelength band MB may belong to the first wavelength band. The light source 30 in which at least a part of the main emission wavelength band belongs to the first wavelength band includes, for example, a light source in which the upper limit of the main emission wavelength band MB exceeds 235 nm. Examples of the light source having the upper limit of the main emission wavelength band MB exceeding 235 nm include solid state light sources such as LEDs.

Fig. 4 is a cross-sectional view of the ultraviolet irradiation device 1 in Fig. 1 as viewed in the X direction. An optical axis Lc of the ultraviolet light L1 emitted from the ultraviolet irradiation device 1 is shown together with an arrow indicating an emission direction. The optical axis Lc extends along the Z direction. An optical filter 40 is disposed in the light extraction part 20. The ultraviolet irradiation device 1 emits only light that has passed through the optical filter 40 among ultraviolet light having a spectrum as shown in Fig. 3.

In a case where the light source 30 is an excimer lamp extending in one direction (Y direction in this example) as shown in Figs. 2 and 4, the light distribution angle of the emitted light flux appearing on a plane (for example, YZ plane) including the extending direction may be different from the light distribution angle of the emitted light flux appearing on a plane (for example, XZ plane) orthogonal to the extending direction. In the present specification, in a case where the light intensity for each light distribution angle is expressed as an angular distribution of light, for the sake of convenience, the "angular distribution of light" is defined by using an arithmetic mean value of the light intensity for each light distribution angle appearing in a plane including the extending direction and for the light intensity for each light distribution angle appearing in a plane orthogonal to the extending direction.

[Optical filter]

The optical filter 40 mainly transmits light in the first wavelength band (200 nm to 235 nm) and mainly reflects light belonging to the second wavelength band (240 nm to 280 nm). The optical filter 40 of the present embodiment transmits the entire main emission wavelength band MB. However, the optical filter 40 only needs to transmit at least a part of the main emission wavelength band MB.

For a spectrum of light transmitted through the optical filter 40 and emitted from the ultraviolet irradiation device 1, a value obtained by integrating light intensity with a wavelength is referred to as a first integrated light intensity, wherein the light intensity is integrated in the first wavelength band overlapping the main emission wavelength band MB and the first wavelength band (200 nm to 235 nm). In the present embodiment in which the entire main emission wavelength band MB (216 nm or more and 223 nm or less) is in the first wavelength band, a value obtained by integrating the light intensity of the main emission wavelength band MB with a wavelength corresponds to the first integrated light intensity. A value obtained by integrating light intensity in the entire second wavelength band (240 nm to 280 nm) with a wavelength is referred to as a second integrated light intensity.

When the maximum value (for example, in a case where the emission angle of the optical filter 40 is 0°) of the first integrated light intensity is compared with the second integrated light intensity, the second integrated light intensity is very small with respect to the maximum value of the first integrated light intensity. Specifically, the second integrated light intensity is 3% or less, more preferably 2% or less, and still more preferably 1% or less to the maximum value of the first integrated light intensity.

The optical filter 40 includes a dielectric multilayer film formed on a base material. Examples of the dielectric multilayer film include a dielectric multilayer film in which hafnium oxide ($HfO_2$) layers and silicon dioxide ($SiO_2$) layers are alternately laminated, and a dielectric multilayer film in which $SiO_2$ layers and aluminum oxide ($Al_2O_3$) layers are alternately laminated. The dielectric multilayer film in which the $HfO_2$ layers and the $SiO_2$ layers are alternately laminated can reduce the number of layers for obtaining the same wavelength-selective characteristics as compared with the dielectric multilayer film in which the $SiO_2$ layers and the $Al_2O_3$ layers are alternately laminated, and thus can increase the transmittance of the selected ultraviolet light.

A base material of the optical filter 40 is made of material that can transmit ultraviolet light included in the first wavelength band of 200 nm to 235 nm. As the specific material for the base material, the following can be adopted which is a ceramic-based material such as silica glass, borosilicate glass, sapphire, magnesium fluoride material, calcium fluoride material, lithium fluoride material, and barium fluoride material, or a resin-based material such as a silicon resin and a fluororesin.

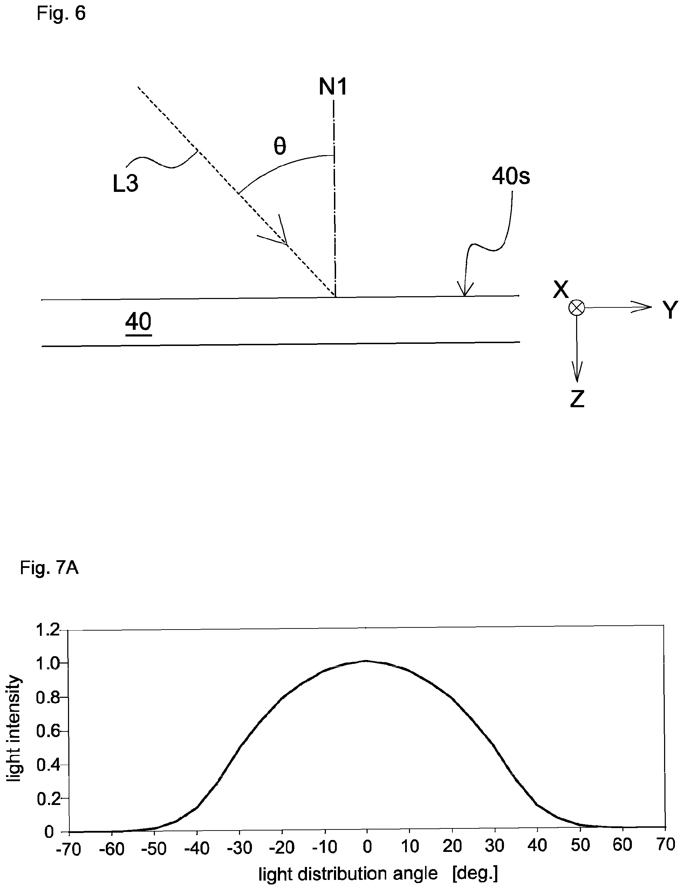

The transmittance of the ultraviolet light transmitted through the optical filter 40 changes depending on an incident angle $\theta$ incident on the optical filter 40 and a wavelength band range of the ultraviolet light to be transmitted. As shown in Fig. 6, the incident angle $\theta$ is an angle formed between a light beam L3 incident on the optical filter 40 and a normal line N1 of an incident surface 40s of the optical filter 40 on which the light beam L3 is incident.

As an evaluation method of transmittance characteristics of the optical filter 40, there is a method of obtaining and using an average transmittance in a specific wavelength band according to the incident angle. The average transmittance in a specific wavelength band is obtained from a transmission spectrum $T(\lambda,\theta)$ for each incident angle of the optical filter 40. A method of obtaining the transmission spectrum T(λ,θ) for each incident angle is described later.

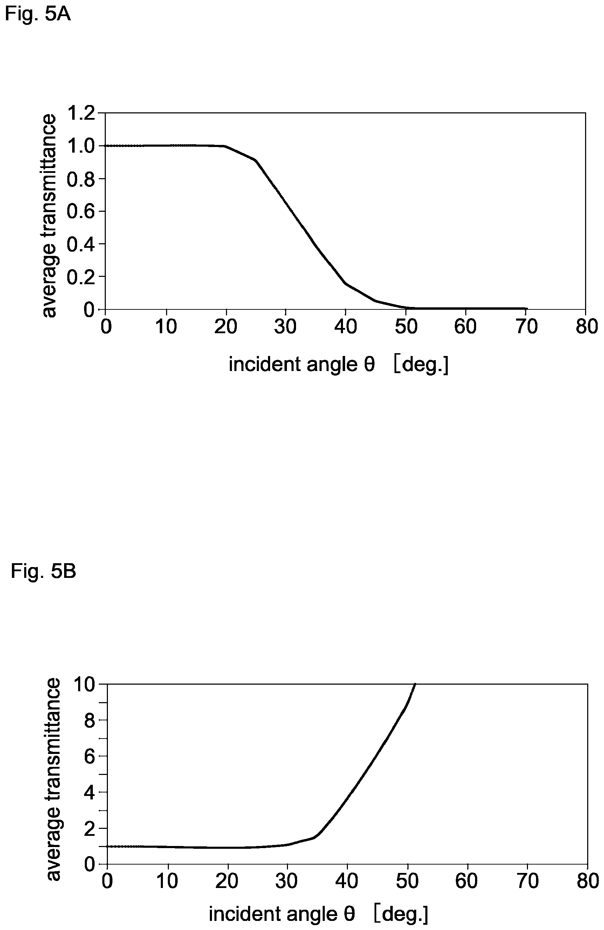

Figs. 5A and 5B show results of obtaining the transmittance characteristics of the optical filter 40 by the above-described evaluation method. Fig. 5A shows incident angle characteristics of the average transmittance in the main emission wavelength band MB (in the present embodiment, 216 nm or more and 223 nm or less) that is transmitted through the optical filter 40. In the graph in Fig. 5A, the horizontal axis represents the incident angle θ at which the light of the main emission wavelength band MB is incident on the optical filter 40, and the vertical axis represents the average transmittance of the main emission wavelength band MB. The transmittance is represented by a relative value in which the transmittance is 1 when an incident angle of light of the main emission wavelength band MB is 0 degrees.

Fig. 5B shows incident angle characteristics of the average transmittance of the harmful light (240 nm to 280 nm) transmitted through the optical filter 40. Although by much smaller amount than the amount of the target light, the optical filter 40 transmits the harmful light. In the graph in Fig. 5B, the horizontal axis represents the incident angle θ at which the harmful light is incident on the optical filter 40, and the vertical axis represents the average transmittance of the harmful light. The transmittance is expressed as a relative value in which the transmittance is 1 when an incident angle of the harmful light is 0 degrees.

From Figs. 5A and 5B, the average transmittance of the target light tends to decrease as the incident angle θ on the optical filter 40 increases from 20 degrees to 60 degrees. In contrast to the transmission characteristics of the target light, the average transmittance of the harmful light tends to increase as the incident angle θ on the optical filter 40 increases from 30 degrees to 60 degrees. As shown in Figs. 17A and 17B, the difference in the transmission characteristics with respect to the incident angle θ caused by the difference in the wavelength band becomes a factor that the angular distribution of the light intensity is different between the target light and the harmful light.

[Light angular distribution of target light/harmful light]

Fig. 7A is an angular distribution of target light emitted from the ultraviolet irradiation device 1 of the present embodiment. More specifically, the angular distribution of the target light is a diagram obtained from a spectrum for each light distribution angle of the light emitted from the optical filter 40 by integrating light intensity of a wavelength band with a wavelength and showing the integrated light intensity for each light distribution angle, the wavelength band including the main emission wavelength band MB that overlaps the first wavelength band. This is referred to as a first angular distribution of light. In the case of the present embodiment in which the entire main emission wavelength band MB (216 nm or more and 223 nm or less) is in the first wavelength band, the first angular distribution of light is a diagram obtained by integrating the light intensity of the main emission wavelength band MB with a wavelength and showing the integrated light intensity for each light distribution angle.

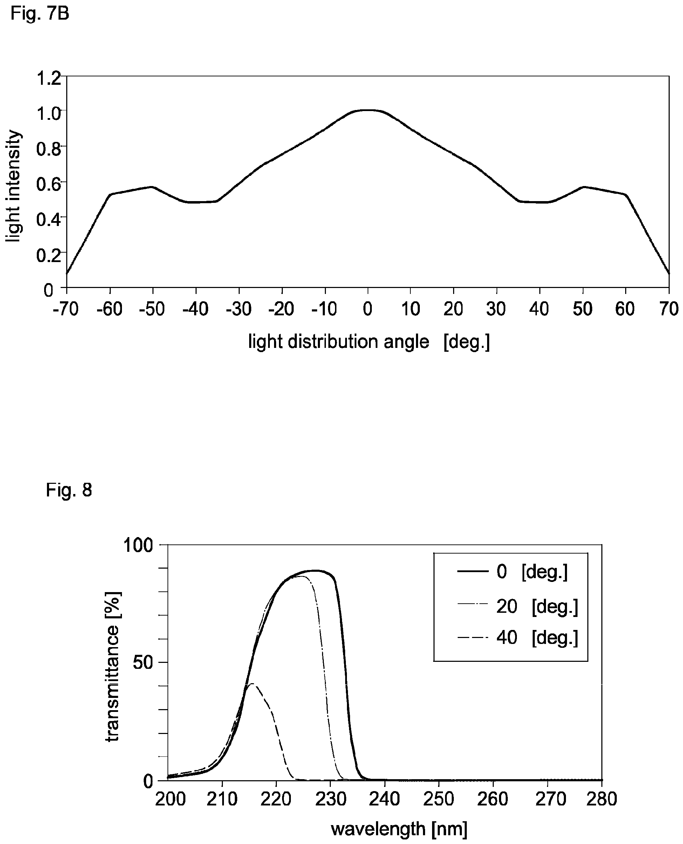

Fig. 7B is an angular distribution of the harmful light emitted from the ultraviolet irradiation device 1 of the present embodiment. More specifically, this is an angular distribution of light obtained from a spectrum for each light distribution angle of light emitted from the optical filter 40 by integrating light intensity in the second wavelength band (240 nm to 280 nm) with a wavelength for each light distribution angle. This is referred to as a second angular distribution of light. The first angular distribution of light and the second angular distribution of light are obtained by a method described later.

In each of the first angular distribution of light and the second angular distribution of light, the horizontal axis represents the light distribution angle (deg.), and the vertical axis represents the light intensity I(θ). Note that it should be understood that the light distribution angle (deg.) and the light intensity I(θ) are similarly defined as in Figs. 17A and 17B, which are described with reference to Figs. 18 and 19.

From the first angular distribution of light in Fig. 7A, it can be seen that the light distribution angle of the light beam indicating the maximum intensity of the target light is 0 degrees. From Fig. 7B, it can be seen that the light distribution angle of the light beam indicating the maximum intensity of the harmful light is 0 degrees. That is, both the maximum intensity of the target light and the maximum intensity of the harmful light appear on the optical axis Lc of the light emitted from the ultraviolet irradiation device 1. Therefore, when the irradiation dose D according to the relaxation of the TLV is set, the irradiation dose D of both the target light and the harmful light can be monitored with the spectroscope or the optical sensor by only measuring the light intensity (relative irradiance) at the position of the optical axis Lc on the irradiated surface.

As both the light distribution angle (first angle) of the light beam indicating the maximum intensity in the first angular distribution of light and the light distribution angle (second angle) of the light beam indicating the maximum intensity in the second angular distribution of light become closer to 0 degrees, the positions (P1, P2) where the light intensity is monitored can be set in the vicinity of the optical axis Lc of the emitted light. As a result, the adjustment of the ultraviolet irradiation device 1 according to the relaxation of the TLV becomes simple.

According to Fig. 7A, as the absolute value of the light distribution angle gradually increases from the first angle (in the example in Fig. 7A, near 0 degrees), 40 degrees, and to 70 degrees, the light intensities of the target light and the harmful light decrease. According to Fig. 7B, as the absolute value of the light distribution angle gradually increases from the second angle (in the example in Fig. 7B, near 0 degrees), 40 degrees, and to 70 degrees, the light intensities of the target light and the harmful light decrease. This leads to the fact that there is no problem even when a light beam having a light distribution angle of 40 degrees or 70 degrees is emitted as long as the light intensities at the first angle and the second angle are considered at the time of determining the irradiation dose D according to the relaxation of the TLV. Therefore, the adjustment of the ultraviolet irradiation device according to the relaxation of the TLV becomes simple.

Further, according to Figs. 7A and 7B, as the absolute value of the light distribution angle increases from the first angle or the second angle to 30 degrees, the light intensities of the target light and the harmful light gradually decrease. This leads to the fact that there is no problem in the irradiated surface irradiated with a light beam having a light distribution angle of $\pm 30$ degrees from the first angle (second angle) as long as the light intensity at the first angle or the second angle is considered at the time of determining the irradiation dose D according to the relaxation of the TLV. Therefore, the adjustment of the ultraviolet irradiation device according to the relaxation of the TLV becomes simple.

[Method of matching second angle with first angle]

As shown in Figs. 7A and 7B, regarding the emitted light emitted from the optical filter 40, the second angle indicating the maximum value of the integrated light intensity in the second angular distribution of light obtained by integrating with a wavelength of the second wavelength band is substantially matched with the first angle indicating the maximum value of the integrated light intensity in the first angular distribution of light obtained by integrating the light intensity of the main emission wavelength band. Therefore, there are roughly two methods, which are: (I) a method of adjusting the incident angle incident on the optical filter 40; and (II) a method of using the optical filter 40 having appropriate transmittance characteristics according to the incident angle. The first angle and the second angle can be substantially matched with each other by at least one of the method (I) and the method (II).

The method (I) of adjusting the incident angle incident on the optical filter 40 is described. When the light distribution angle of the emitted light from the light source 30 is small, the target light is easily transmitted and the harmful light is not easily transmitted. In the light source 30, for example, the absolute value of the light distribution angle that is a half value of the maximum intensity of the emitted light is preferably within 60 degrees, and more preferably within 40 degrees.

An example of a method of adjusting the light distribution angle of the light source 30 is described. For example, in a case where the surfaces of the pair of electrodes 30b function as reflecting surfaces of emitted light from the light-emitting tubes 30a, the incident angle incident on the optical filter 40 can be adjusted by adjusting the shape of each of the electrodes 30b.

The incident angle at which the emitted light from the light source 30 is incident on the optical filter 40 may be adjusted by arranging a transmission optical system such as a lens between the light source 30 and the optical filter 40. In addition, the incident angle incident on the optical filter 40 may be adjusted by changing the shape of the light source 30 itself.

In the method (II) of using the optical filter 40 having appropriate transmittance characteristics, in the optical filter 40, the transmittance characteristics according to the incident angle change when a composition, a layer thickness, the number of laminated layers, and the like of the dielectric multilayer film change. An intrinsic angular distribution of light is determined by the transmittance characteristics according to the incident angle. Therefore, by obtaining the angular distribution of light, the optical filter 40 having desired transmittance characteristics according to the incident angle is designed or selected.

[Method of obtaining angular distribution of light]

An example of a method of obtaining the angular distribution of light is described. The light angular distribution of light of the optical filter 40 can be obtained by performing the following steps (a) to (e). As described above, after the angular distribution of light is obtained, the first angle of the light beam indicating the maximum value of the integrated light intensity in the first angular distribution of light is compared with the second angle of the light beam indicating the maximum value of the integrated light intensity in the second angular distribution of light. Then, depending on whether the difference between the first angle and the second angle is small or not, the transmittance characteristics according to the incident angle of the optical filter 40 is determined to be appropriate or not.

(a) Measurement of a spectrum of light emitted from the light source 30

Light intensity $I(\lambda)$ emitted from the light source 30 is measured using a spectroscope to obtain a spectrum. In this measurement, for example, the emitted light from the ultraviolet irradiation device 1 may be measured in a state where the optical filter 40 is removed from the ultraviolet irradiation device 1. The wavelength to be measured is in a wavelength range (in the present embodiment, a range from 200 nm to 280 nm) including the target light and the harmful light. The measurement result is shown as, for example, a graph in which the wavelength (nm) is plotted on the horizontal axis and the relative intensity with the intensity of the wavelength showing the maximum intensity as 100% is plotted on the vertical axis as shown in Fig. 3.

(b) Measurement of a transmission spectrum of the optical filter 40 for each incident angle

The optical filter 40 to be a candidate is prepared, and the transmission spectrum $T(\lambda,\theta)$ of the optical filter 40 alone for each incident angle is obtained. For example, as shown in Fig. 8, the transmission spectrum $T(\lambda,\theta)$ for each incident angle is shown as a graph in which the wavelength (nm) is plotted on the horizontal axis and the transmittance (%) is plotted on the vertical axis. Fig. 8 shows a total of three transmission spectra $T(\lambda,\theta)$ when the incident angle $\theta$ of the optical filter 40 is 0 degrees, 20 degrees, and 40 degrees.

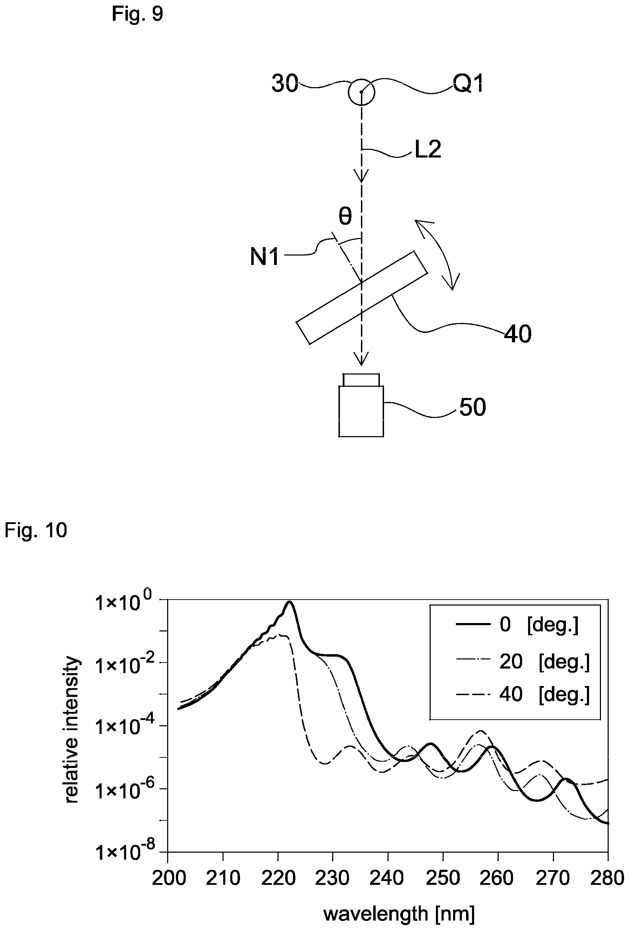

Fig. 9 shows an example of a measurement method of obtaining the transmission spectrum $T(\lambda,\theta)$ for each incident angle. The optical filter 40 inclined at a predetermined incident angle $\theta$ with respect to the normal line N1 of the optical filter 40 is arranged. Then, directional light L2 is made incident on the optical filter 40 from a center Q1 of the light source 30. The light intensity of the light transmitted through the optical filter 40 is measured by a spectroscope 50. By removing light intensity of the emitted light in a case where there is no optical filter from the measured light intensity, the transmission spectrum $T(\lambda)$ of the optical filter 40 is obtained. Then, the transmission spectrum $T(\lambda,\theta)$ is obtained by performing measurement while changing the incident angle $\theta$ by inclining the optical filter 40 with respect to the traveling direction of the light L2.

Fig. 8 only shows a total of three transmission spectra $T(\lambda,\theta)$ of the incident angles at 20 degree intervals, which are 0 degrees, 20 degrees, and 40 degrees. However, the interval for obtaining the transmission spectrum $T(\lambda, \theta)$ may be narrowed. For example, the measurement may be performed while changing the incident angle by 5 degrees between 0 to 70 degrees to obtain a total of 15 transmission spectra $T(\lambda,\theta)$ having different incident angles $\theta$.

(c) Calculation of the transmission spectrum of the emitted light of the optical filter for each incident angle

By multiplying the light intensity $I(\lambda)$ obtained in (a) by the transmission spectrum $T(\lambda,\theta)$ of the optical filter 40 obtained in (b), light intensity $I_{FO}(\lambda,\theta)$ of the emitted light transmitted through the optical filter 40 for each incident angle is obtained. That is, the light intensity $I_{FO}(\lambda,\theta)$ of the light emitted from the optical filter 40 for each incident angle is expressed by the following formula (1).
[Formula 1]

$$I_{FO}\big(\lambda, \; \theta\big) \;\; = \;\; I(\lambda) \times T\big(\lambda, \; \theta\big) \quad \dots \; (1)$$

In Fig. 10, the spectrum of the light intensity $I_{FO}(\lambda,\theta)$ emitted from the optical filter 40 is shown for each incident angle (as an example, three types of incident angles are shown, which are 0 degrees, 20 degrees, and 40 degrees) incident on the optical filter 40.

(d) Calculation of integrated intensity of the harmful light

By using the light intensity $I_{FO}(\lambda,\theta)$ of the emitted light obtained in (c), integrated intensity $S_{B2}(\theta)$ of the second wavelength band (240 to 280 nm) indicating the harmful light is obtained. The integrated intensity $S_{B2}(\theta)$ of the second wavelength band is expressed by the following formula (2).
[Formula 2]

$$S_{B2}(\theta) \;\; = \;\; \int_{240}^{280} I_{FO}\big(\lambda, \; \theta\big) d\lambda$$

$$= \;\; \int_{240}^{280} I(\lambda) \times T\;(\lambda, \; \theta) \; d\lambda \quad \dots \; (2)$$

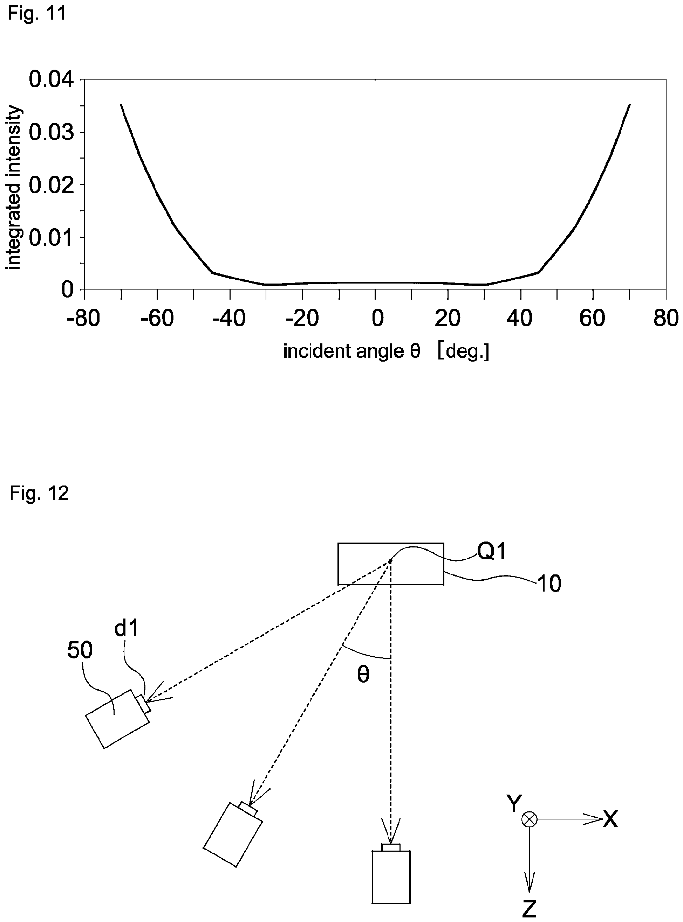

Fig. 11 shows incident angle characteristics of the optical filter 40 in the second wavelength band. By obtaining the integrated intensity $S_{B2}(\theta)$ of the second wavelength band for the incident angle of 0 to 70 degrees, for example, a graph in which the incident angle $\theta$ to the optical filter 40 is plotted on the horizontal axis and the integrated intensity (relative value) of the second wavelength band is plotted on the vertical axis as in Fig. 11 is obtained. As can be seen from the graph of Fig. 11, the integrated intensity $S_{B2}(\theta)$ of the second wavelength band increases as the absolute value of the incident angle $\theta$ on the optical filter 40 increases.

(e) Calculation of an angular distribution of the emitted light from an ultraviolet irradiation device without the optical filter 40

An angular distribution of the light emitted from an ultraviolet irradiation device 10 without the optical filter 40 is obtained by the following procedure. First, for example, as shown in Fig. 12, while the spectroscope 50 is rotationally moved about the center Q1 of the light source 30 so as to draw a circle around the Y-axis direction, the radiation intensity of the emitted light is measured by the spectroscope 50. As a result, relative radiation intensity $E(\theta)$ for each light distribution angle of the ultraviolet irradiation device 10 can be obtained. Then, from the relative radiation intensity $E(\theta)$, the light intensity $I(\theta)$ of the emitted light from the ultraviolet irradiation device 10 on the irradiated plane can be obtained (see Fig. 1). The light intensity $I(\theta)$ is obtained by the following formula (3).

[Formula 3]

$$I(\theta) \;=\; E(\theta) \times (cos\theta)^3 \quad \ldots \;(3)$$

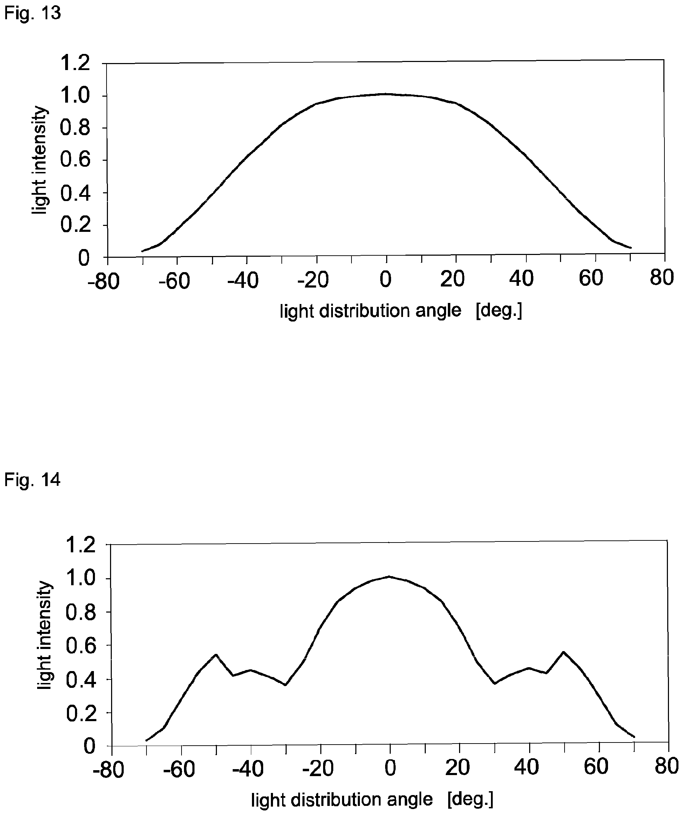

For example, as shown in Fig. 13, the light intensity $I(\theta)$ of the emitted light from the ultraviolet irradiation device 10 without the optical filter 40 is indicated as the angular distribution of light in which the incident angle (light distribution angle) $\theta$ to the optical filter 40 is plotted on the horizontal axis and the relative value of the light intensity in a case where the light intensity at $\theta = 0$ is 1 is plotted on the vertical axis.

By multiplying the light intensity $I(\theta)$ by the integrated intensity $S_{B2}(\theta)$ in the second wavelength band, a second angular distribution $I_{B2}(\theta)$ that is a light intensity distribution according to the light distribution angle of the harmful light emitted from the ultraviolet irradiation device 1 is obtained. That is, the second angular distribution $I_{B2}(\theta)$ of the harmful light is obtained by the following formula (4).

[Formula 4]

$$I_{B2}(\theta) \;=\; I(\theta) \times S_{B2}(\theta)$$

$$=\; E(\theta) \times (cos\theta)^3 \times \int_{240}^{280} I(\lambda) \times T\;(\lambda,\;\theta)\;d\lambda \quad \ldots \;(4)$$

Fig. 14 shows the second angular distribution $I_{B2}(\theta)$ in which the light distribution angle (deg.) is plotted on the horizontal axis, and the relative value of light intensity obtained by integrating the wavelength in a range of 240 nm to 280 nm is plotted on the vertical axis.

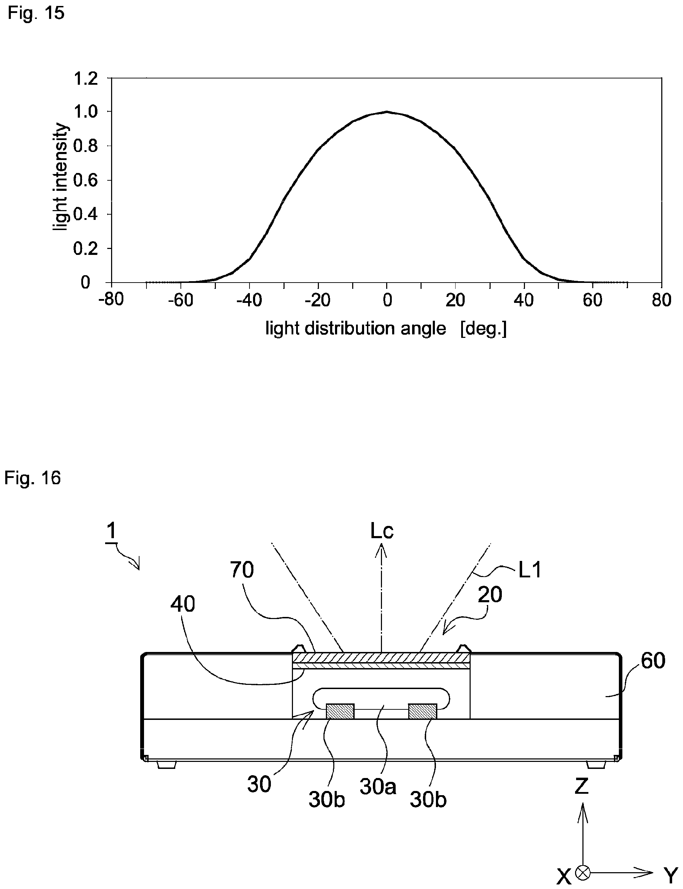

The method of obtaining the angular distribution of light has been described above by taking the harmful light as an example. A first angular distribution $I_{B1}(\theta)$ of the target light can be obtained by the similar method as described above. Fig. 15 shows the first light distribution angle distribution $I_{B1}(\theta)$ in which the light distribution angle (deg.) is plotted on the horizontal axis and the integrated intensity of the target light is plotted on the vertical axis.

Comparison between the second angular distribution $I_{B2}(\theta)$ in Fig. 14 and the first angular distribution $I_{B1}(\theta)$ in Fig. 15 shows that the difference is small between the first angle of the light beam indicating the maximum value of the integrated light intensity in the first light distribution angle distribution and the second angle of the light beam indicating the maximum value of the integrated light intensity in the second angular distribution of light, and that the optical filter 40 has appropriate transmittance characteristics for the ultraviolet irradiation device.

The embodiment of the ultraviolet irradiation device has been described above. However, the present invention is not limited to the above embodiment, and various changes or modifications may be made to the above embodiment without departing from the spirit of the present invention.

As an improvement example, as shown in Fig. 16, a diffusion part 70 that diffuses the emitted light of the optical filter 40 may be provided at a subsequent stage of the optical filter 40. This makes it possible to spread the ultraviolet light over a wide range in a suitable state. In a case where the diffusion part 70 is provided, the effect obtained by the present invention is more remarkable.

More specifically, in the conventional ultraviolet irradiation device shown in Figs. 17A and 17B, the position P1 irradiated with the light beam indicating the maximum intensity of the target light differs greatly from the position P2 irradiated with the light beam indicating the maximum intensity of the harmful light (see Fig. 19). Therefore, in a case of using the ratio of the harmful light to the target light at the position P1 as a reference, when the emitted light from the optical filter 40 is diffused by the diffusion part 70, the ratio of the harmful light included in the diffused light is averaged to cause the ratio of the harmful light to be easily increased more than the reference. In addition, the degree of the increase rate is also difficult to be predicted.

On the other hand, in the present invention, as described above, both the light distribution angle (first angle) of the light beam indicating the maximum intensity in the first light distribution angle distribution and the light distribution angle (second angle) of the light beam indicating the maximum intensity in the second angular distribution of light become closer to 0 degrees. That is, the position P1 irradiated with the light beam indicating the maximum intensity of the target light becomes close to the position P2 irradiated with the light beam indicating the maximum intensity of the harmful light. Therefore, when the emitted light from the optical filter 40 is diffused by the diffusion part 70, the ratio of the harmful light included in the diffused light maintains the reference or is reduced to be lower than the reference. Therefore, management in terms of safety is easier, and the ultraviolet light can be spread in a wide range in a more preferable state. The diffusion part 70 is, for example, a plate-like or film-like optical member.

The optical filter 40 may be disposed in the vicinity of the light source 30 instead of being disposed in the light extraction part 20. In addition, the optical filter 40 may be disposed as a part of the light source 30, for example, in a sealing body of the lamp.

DESCRIPTION OF REFERENCE NUMERALS

| | |
|---|---|
| 1, 100 | Ultraviolet irradiation device |
| 10 | Ultraviolet irradiation device (without optical filter) |
| 20 | Light extraction part |
| 30 | Light source |
| 30a | Light-emitting tube |
| 30b | Electrode |
| 40 | Optical filter |
| 50 | Spectroscope |
| 60 | Casing |
| 70 | Diffusion part |
| 90 | Irradiated plane |

**Claims**

1. An ultraviolet irradiation device (1) comprising:

   a light source (30) that emits light showing light intensity in a first wavelength band of 200 nm to 235 nm and a second wavelength band of 240 nm to 280 nm and having at least a part of a main emission wavelength band belonging to the first wavelength band;
   an optical filter (40) that suppresses the light intensity in the second wavelength band and has transmittance characteristics according to an incident angle of the light emitted from the light source (30), wherein,
   regarding a first angular distribution of light obtained from a spectrum for each light distribution angle of the light emitted from the optical filter (40) by integrating the light intensity with a wavelength and showing an integrated value for each light distribution angle, the light intensity being in a wavelength band where the main emission wavelength band overlaps the first wavelength band, and
   regarding a second angular distribution of light obtained from the spectrum for each light distribution angle by integrating the light intensity in the second wavelength band with a wavelength and showing an integrated value for each light distribution angle,
   the first angular distribution of light has a first angle of a light beam showing maximum intensity, the first angle substantially matching with a second angle of a light beam showing maximum intensity in the second angular distribution of light.

2. The ultraviolet irradiation device (1) according to claim 1, wherein the first angle and the second angle are both between -10 degrees and + 10 degrees.

3. The ultraviolet irradiation device (1) according to claim 2, wherein,

   in the first angular distribution of light, the light intensity of the light beam having a light distribution angle of 40 degrees is smaller than the light intensity of the light beam having the first angle, and the light intensity of the light beam having a light distribution angle of 70 degrees is smaller than the light intensity of the light beam having the light distribution angle of 40 degrees, and

in the second angular distribution of light, the light intensity of the light beam having a light distribution angle of 40 degrees is smaller than the light intensity of the light beam having the second angle, and the light intensity of the light beam having a light distribution angle of 70 degrees is smaller than the light intensity of the light beam having the light distribution angle of 40 degrees.

4. The ultraviolet irradiation device (1) according to any one of claims 1 to 3, wherein, in a spectrum of light emitted from the optical filter (40), regarding a first integrated light intensity obtained by integrating the light intensity of a wavelength band in which the main emission wavelength band overlaps the first wavelength band and a second integrated light intensity obtained by integrating the light intensity in the second wavelength band, the second integrated light intensity is 1.0% or less of the first integrated light intensity.

5. The ultraviolet irradiation device (1) according to claim 4, wherein the second integrated light intensity is 0.1% or less of the first integrated light intensity.

6. The ultraviolet irradiation device (1) according to any one of claims 1 to 3, wherein the optical filter (40) includes, at a subsequent stage, a diffusion part that diffuses emitted light of the optical filter (40).

7. The ultraviolet irradiation device (1) according to any one of claims 1 to 3, wherein

the optical filter (40) has an average transmittance for light in a wavelength band in which the main emission wavelength band overlaps the first wavelength band, the average transmittance showing a decreasing tendency as the incident angle on the optical filter (40) increases from 20 degrees to 60 degrees, and

the optical filter (40) has the average transmittance of the optical filter (40) for the light in the second wavelength band, the average transmittance showing an increasing tendency as the incident angle on the optical filter (40) increases from 30 degrees to 60 degrees.

Fig. 1

Fig. 2

Fig. 3

Fig. 4

Fig. 5A

Fig. 5B

Fig. 6

Fig. 7A

Fig. 7B

Fig. 8

Fig. 9

Fig. 10

Fig. 11

Fig. 12

Fig. 13

Fig. 14

Fig. 15

Fig. 16

Fig. 17A

Fig. 17B

Fig. 18

Fig. 19

Fig. 20

Europäisches
Patentamt

European
Patent Office

Office européen
des brevets

# EUROPEAN SEARCH REPORT

Application Number

EP 23 16 3617

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/149406 A1 (USHIO ELECTRIC INC [JP]) 29 July 2021 (2021-07-29) * the whole document * & EP 4 095 884 A1 (USHIO ELECTRIC INC [JP]) 30 November 2022 (2022-11-30) * the whole document * | 1-3,6,7 | INV. A61L9/20 A61N5/06 |
| X | JP 7 015485 B1 (USHIO ELECTRIC INC) 3 February 2022 (2022-02-03) * the whole document * | 1,4-7 | |
| X,P | EP 4 008 361 A1 (USHIO ELECTRIC INC [JP]) 8 June 2022 (2022-06-08) * the whole document * | 1-7 | |

TECHNICAL FIELDS
SEARCHED      (IPC)

A61L
A61N

The present search report has been drawn up for all claims

| Place of search | Date of completion of the search | Examiner |
|---|---|---|
| Munich | 25 July 2023 | Beck, Ewa |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

.................................................................

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 23 16 3617

This annex lists the patent family members relating to the patent documents cited in the above-mentioned European search report.
The members are as contained in the European Patent Office EDP file on
The European Patent Office is in no way liable for these particulars which are merely given for the purpose of information.

25-07-2023

| Patent document cited in search report | | Publication date | Patent family member(s) | | Publication date |
|---|---|---|---|---|---|
| WO 2021149406 | A1 | 29-07-2021 | CN | 114787963 A | 22-07-2022 |
| | | | EP | 4095884 A1 | 30-11-2022 |
| | | | JP | 2021114423 A | 05-08-2021 |
| | | | US | 2023054791 A1 | 23-02-2023 |
| | | | WO | 2021149406 A1 | 29-07-2021 |
| JP 7015485 | B1 | 03-02-2022 | JP | 7015485 B1 | 03-02-2022 |
| | | | JP | 2022170807 A | 11-11-2022 |
| | | | WO | 2022230359 A1 | 03-11-2022 |
| EP 4008361 | A1 | 08-06-2022 | CN | 114630686 A | 14-06-2022 |
| | | | EP | 4008361 A1 | 08-06-2022 |
| | | | KR | 20230049711 A | 13-04-2023 |
| | | | WO | 2022074944 A1 | 14-04-2022 |

EPO FORM P0459

For more details about this annex : see Official Journal of the European Patent Office, No. 12/82

**REFERENCES CITED IN THE DESCRIPTION**

**Patent documents cited in the description**

• JP 6908172 B